# EUROPEAN PATENT APPLICATION

(11) **EP 1 357 182 A1**
(43) Date of publication of application: **29.10.2003**
(21) Application number: 01994988.2
(22) Date of filing: 21.12.2001
(51) Int. Cl.: C12N 15/09, C12N 9/90, C12P 19/28

(54) **PROCESS FOR PRODUCING UDP-N-ACETYLGALACTOSAMINE AND SACCHARIDE CONTAINING N-ACETYLGALACTOSAMINE**

(30) Priority: 21.12.2000 JP 2000388992
(71) Applicant: Kyowa Hakko Kogyo Co., Ltd, Tokyo 100-8185 (JP)
(72) Inventor: ENDO, Tetsuo, c/o Tokyo Research Laboratories, Machida-shi, Tokyo 194-8533 (JP); KOIZUMI, Satoshi, c/o Tokyo Research Laboratories, Machida-shi, Tokyo 194-8533 (JP); TABATA, Kazuhiko, c/o Tech. Research Laboratories, Hofu-sshi, Yamaguchi 747-8522 (JP); OZAKI, Akio, c/o Tech. Research Laboratories, Hofu-shi, Yamaguchi 747-8522 (JP)
(74) Representative: VOSSIUS & PARTNER
(86) International application number: JP0111269
(87) International publication number: WO02050267

(57) **Abstract**

According to the present invention, UDP-N-acetylgalactosamine and an N-acetylgalactosamine-containing carbohydrate can be produced using a protein having UDP-N-acetylglucosamine 4-epimerase activity.

SEQUENCE LISTING

<110> KYOWA HAKKO KOGYO CO., LTD.

<120> Process for the production of UDP-N-acetylgalactosamine and carboh
ydrates containing N-acetylgalactosamine

<130> H12-1411A4

<140>
<141>

<150> JP 2000-388992
<151> 2000-12-21

<160> 14

<170> PatentIn Ver. 2.1

<210> 1
<211> 339
<212> PRT
<213> Bacillus subtilis

<400> 1
Met Ala Ile Leu Val Thr Gly Gly Ala Gly Tyr Ile Gly Ser His Thr
  1               5                  10                  15

Cys Val Glu Leu Leu Asn Ser Gly Tyr Glu Ile Val Val Leu Asp Asn
             20                  25                  30

Leu Ser Asn Ser Ser Ala Glu Ala Leu Asn Arg Val Lys Glu Ile Thr
         35                  40                  45

Gly Lys Asp Leu Thr Phe Tyr Glu Ala Asp Leu Leu Asp Arg Glu Ala
     50                  55                  60

Val Asp Ser Val Phe Ala Glu Asn Glu Ile Glu Ala Val Ile His Phe
 65                  70                  75                  80

Ala Gly Leu Lys Ala Val Gly Glu Ser Val Ala Ile Pro Leu Lys Tyr
                 85                  90                  95

Tyr His Asn Asn Leu Thr Gly Thr Phe Ile Leu Cys Glu Ala Met Glu
            100                 105                 110

Lys Tyr Gly Val Lys Lys Ile Val Phe Ser Ser Ser Ala Thr Val Tyr
        115                 120                 125

Gly Val Pro Glu Thr Ser Pro Ile Thr Glu Asp Phe Pro Leu Gly Ala
    130                 135                 140

Thr Asn Pro Tyr Gly Gln Thr Lys Leu Met Leu Glu Gln Ile Leu Arg
145                 150                 155                 160

Asp Leu His Thr Ala Asp Asn Glu Trp Ser Val Ala Leu Leu Arg Tyr
                165                 170                 175

Phe Asn Pro Phe Gly Ala His Pro Ser Gly Arg Ile Gly Glu Asp Pro
            180                 185                 190

Asn Gly Ile Pro Asn Asn Leu Met Pro Tyr Val Ala Gln Val Ala Val
        195                 200                 205

Gly Lys Leu Glu Gln Leu Ser Val Phe Gly Asn Asp Tyr Pro Thr Lys
    210                 215                 220

Asp Gly Thr Gly Val Arg Asp Tyr Ile His Val Val Asp Leu Ala Glu
225                 230                 235                 240

Gly His Val Lys Ala Leu Glu Lys Val Leu Asn Ser Thr Gly Ala Asp
                245                 250                 255

Ala Tyr Asn Leu Gly Thr Gly Thr Gly Tyr Ser Val Leu Glu Met Val
            260                 265                 270

Lys Ala Phe Glu Lys Val Ser Gly Lys Glu Val Pro Tyr Arg Phe Ala
        275                 280                 285

Asp Arg Arg Pro Gly Asp Ile Ala Thr Cys Phe Ala Asp Pro Ala Lys
    290                 295                 300

Ala Lys Arg Glu Leu Gly Trp Glu Ala Lys Arg Gly Leu Glu Glu Met
305                 310                 315                 320

Cys Ala Asp Ser Trp Arg Trp Gln Ser Ser Asn Val Asn Gly Tyr Lys

325                 330                 335

Ser Ala Glu
        339

<210> 2
<211> 338
<212> PRT
<213> Neisseria gonorrhoeae

<400> 2
Met Thr Val Leu Ile Thr Gly Gly Thr Gly Phe Ile Gly Ser His Thr
 1               5                  10                  15

Ala Val Ser Leu Val Gln Ser Gly Tyr Asp Ala Val Ile Leu Asp Asn
            20                  25                  30

Leu Cys Asn Ser Ser Ala Ala Val Leu Pro Arg Leu Arg Gln Ile Thr
        35                  40                  45

Gly Arg Asn Ile Pro Phe Tyr Gln Gly Asp Ile Arg Asp Cys Gln Ile
    50                  55                  60

Leu Arg Gln Ile Phe Ser Glu His Glu Ile Glu Ser Val Ile His Phe
65                  70                  75                  80

Ala Gly Leu Lys Ala Val Gly Glu Ser Val Ala Glu Pro Thr Lys Tyr
                85                  90                  95

Tyr Gly Asn Asn Val Tyr Gly Ser Leu Val Leu Ala Glu Glu Met Ala
            100                 105                 110

Arg Ala Gly Val Leu Lys Ile Val Phe Ser Ser Ser Ala Thr Val Tyr
        115                 120                 125

Gly Asp Ala Glu Lys Val Pro Tyr Thr Glu Asp Met Arg Pro Gly Asp
    130                 135                 140

Thr Ala Asn Pro Tyr Gly Ala Ser Lys Ala Met Val Glu Arg Met Leu
145                 150                 155                 160

Thr Asp Ile Gln Lys Ala Asp Pro Arg Trp Ser Val Ile Leu Leu Arg

                165                 170                 175

Tyr Phe Asn Pro Ile Gly Ala His Glu Ser Gly Leu Ile Gly Glu Gln
            180                 185                 190

Pro Asn Gly Val Pro Asn Asn Leu Leu Pro Tyr Ile Cys Gln Val Ala
        195                 200                 205

Ser Gly Arg Leu Pro Gln Leu Ser Val Phe Gly Gly Asp Tyr Pro Thr
    210                 215                 220

Pro Asp Gly Thr Gly Met Arg Asp Tyr Ile His Val Met Asp Leu Ala
225                 230                 235                 240

Glu Gly His Ile Ala Ala Met Lys Ala Lys Gly Gly Val Ala Gly Val
                245                 250                 255

His Leu Phe Asn Leu Gly Ser Gly Arg Ala Tyr Ser Val Leu Glu Ile
            260                 265                 270

Ile Arg Ala Phe Glu Ala Ala Ser Gly Leu His Ile Pro Tyr Arg Ile
        275                 280                 285

Gln Pro Arg Arg Ala Gly Asp Leu Ala Cys Ser Tyr Ala Asp Pro Ser
    290                 295                 300

His Thr Lys Gln Gln Thr Gly Trp Glu Thr Lys Arg Gly Leu Gln Gln
305                 310                 315                 320

Met Met Glu Asp Ser Trp Arg Trp Val Ser Arg Asn Pro Gly Arg Tyr
                325                 330                 335

Gly Asp
338

<210> 3
<211> 1017
<212> DNA
<213> Bacillus subtilis

<400> 3
atg gca ata ctt gtt act ggc ggt gcc ggt tac att ggc agc cac aca 48

Met Ala Ile Leu Val Thr Gly Gly Ala Gly Tyr Ile Gly Ser His Thr
1               5                   10                  15

tgt gtt gaa cta ttg aac agc ggc tac gag att gtt gtt ctt gat aat     96
Cys Val Glu Leu Leu Asn Ser Gly Tyr Glu Ile Val Val Leu Asp Asn
            20                  25                  30

ctg tcc aac agt tca gct gaa gcg ctg aac cgt gtc aag gag att aca     144
Leu Ser Asn Ser Ser Ala Glu Ala Leu Asn Arg Val Lys Glu Ile Thr
        35                  40                  45

gga aaa gat tta acg ttc tac gaa gcg gat tta ttg gac cgg gaa gcg     192
Gly Lys Asp Leu Thr Phe Tyr Glu Ala Asp Leu Leu Asp Arg Glu Ala
    50                  55                  60

gta gat tcc gtt ttt gct gaa aat gaa atc gaa gct gtg att cat ttt     240
Val Asp Ser Val Phe Ala Glu Asn Glu Ile Glu Ala Val Ile His Phe
65                  70                  75                  80

gca ggg tta aaa gca gtc ggc gaa tct gtg gcg att ccc ctc aaa tat     288
Ala Gly Leu Lys Ala Val Gly Glu Ser Val Ala Ile Pro Leu Lys Tyr
                85                  90                  95

tat cat aac aat ttg aca gga acg ttt att tta tgc gag gcc atg gag     336
Tyr His Asn Asn Leu Thr Gly Thr Phe Ile Leu Cys Glu Ala Met Glu
            100                 105                 110

aaa tac ggc gtc aag aaa atc gta ttc agt tca tct gcg aca gta tac     384
Lys Tyr Gly Val Lys Lys Ile Val Phe Ser Ser Ser Ala Thr Val Tyr
        115                 120                 125

ggc gtt ccg gaa aca tcg ccg att acg gaa gac ttt cca tta ggc gcg     432
Gly Val Pro Glu Thr Ser Pro Ile Thr Glu Asp Phe Pro Leu Gly Ala
    130                 135                 140

aca aat cct tat ggg cag acg aag ctc atg ctt gaa caa ata ttg cgt     480
Thr Asn Pro Tyr Gly Gln Thr Lys Leu Met Leu Glu Gln Ile Leu Arg
145                 150                 155                 160

gat ttg cat aca gcc gac aat gag tgg agc gtt gcg ctg ctt cgt tac     528
Asp Leu His Thr Ala Asp Asn Glu Trp Ser Val Ala Leu Leu Arg Tyr
                165                 170                 175

ttt aac ccg ttc ggc gcg cat cca agc gga cgg atc ggt gaa gac ccg 576
Phe Asn Pro Phe Gly Ala His Pro Ser Gly Arg Ile Gly Glu Asp Pro
            180                 185                 190

aac gga atc cca aat aac ctt atg ccg tat gtg gca cag gta gca gtc 624
Asn Gly Ile Pro Asn Asn Leu Met Pro Tyr Val Ala Gln Val Ala Val
        195                 200                 205

ggg aag ctc gag caa tta agc gta ttc gga aat gac tat ccg aca aaa 672
Gly Lys Leu Glu Gln Leu Ser Val Phe Gly Asn Asp Tyr Pro Thr Lys
    210                 215                 220

gac ggg aca ggc gta cgc gat tat att cac gtc gtt gat ctc gca gaa 720
Asp Gly Thr Gly Val Arg Asp Tyr Ile His Val Val Asp Leu Ala Glu
225                 230                 235                 240

ggc cac gtc aag gcg ctg gaa aaa gta ttg aac tct aca gga gcc gat 768
Gly His Val Lys Ala Leu Glu Lys Val Leu Asn Ser Thr Gly Ala Asp
                245                 250                 255

gca tac aac ctt gga aca ggc aca ggc tac agc gtg ctg gaa atg gtc 816
Ala Tyr Asn Leu Gly Thr Gly Thr Gly Tyr Ser Val Leu Glu Met Val
            260                 265                 270

aaa gcc ttt gaa aaa gtg tca ggg aaa gag gtt cca tac cgt ttt gcg 864
Lys Ala Phe Glu Lys Val Ser Gly Lys Glu Val Pro Tyr Arg Phe Ala
        275                 280                 285

gac cgc cgt ccg gga gac atc gcc aca tgc ttt gca gat cct gcg aaa 912
Asp Arg Arg Pro Gly Asp Ile Ala Thr Cys Phe Ala Asp Pro Ala Lys
    290                 295                 300

gcc aag cga gaa cta ggc tgg gaa gcg aaa cgc ggc ctt gag gaa atg 960
Ala Lys Arg Glu Leu Gly Trp Glu Ala Lys Arg Gly Leu Glu Glu Met
305                 310                 315                 320

tgt gct gat tcc tgg aga tgg cag tct tct aat gtg aat ggg tat aag 1008
Cys Ala Asp Ser Trp Arg Trp Gln Ser Ser Asn Val Asn Gly Tyr Lys
                325                 330                 335

agt gcg gaa 1017
Ser Ala Glu

<210> 4
<211> 1014
<212> DNA
<213> Neisseria gonorrhoeae

<400> 4
atg acc gtc ctg att acc ggc ggc acc ggc ttt atc ggt tcg cac acc     48
Met Thr Val Leu Ile Thr Gly Gly Thr Gly Phe Ile Gly Ser His Thr
1               5                   10                  15

gcc gtc tcg ctc gtc caa tcc ggt tac gat gcc gtg att ttg gat aat     96
Ala Val Ser Leu Val Gln Ser Gly Tyr Asp Ala Val Ile Leu Asp Asn
            20                  25                  30

ctg tgc aac tcg tct gcc gcc gtc ctc cca cgc ctt cgg caa att acc     144
Leu Cys Asn Ser Ser Ala Ala Val Leu Pro Arg Leu Arg Gln Ile Thr
        35                  40                  45

ggc aga aac ata ccg ttt tat cag ggc gac atc cgc gac tgt cag att     192
Gly Arg Asn Ile Pro Phe Tyr Gln Gly Asp Ile Arg Asp Cys Gln Ile
    50                  55                  60

ttg agg cag att ttt tca gaa cat gaa atc gaa tcc gtc atc cat ttt     240
Leu Arg Gln Ile Phe Ser Glu His Glu Ile Glu Ser Val Ile His Phe
65                  70                  75                  80

gcc ggt ttg aag gca gtg ggg gaa agc gtt gcc gag ccg aca aaa tat     288
Ala Gly Leu Lys Ala Val Gly Glu Ser Val Ala Glu Pro Thr Lys Tyr
                85                  90                  95

tac ggc aac aat gtt tac ggc agc ctg gtg ctg gcg gaa gaa atg gcg     336
Tyr Gly Asn Asn Val Tyr Gly Ser Leu Val Leu Ala Glu Glu Met Ala
            100                 105                 110

cgc gcg ggc gtg ttg aaa atc gta ttc agc tcg tcg gca acc gtt tac     384
Arg Ala Gly Val Leu Lys Ile Val Phe Ser Ser Ser Ala Thr Val Tyr
        115                 120                 125

ggc gat gcg gaa aaa gtc ccc tat acg gaa gat atg cgc ccg ggc gat     432
Gly Asp Ala Glu Lys Val Pro Tyr Thr Glu Asp Met Arg Pro Gly Asp
    130                 135                 140

acc gct aat cct tac ggt gcg tcc aaa gcg atg gtg gag cgg atg tta 480
Thr Ala Asn Pro Tyr Gly Ala Ser Lys Ala Met Val Glu Arg Met Leu
145                 150                 155                 160

acc gac atc caa aaa gcc gat ccg cgt tgg agc gtg att ttg ttg cgc 528
Thr Asp Ile Gln Lys Ala Asp Pro Arg Trp Ser Val Ile Leu Leu Arg
                165                 170                 175

tat ttc aac ccg atc ggc gcg cac gaa agc gga ctt atc ggc gaa cag 576
Tyr Phe Asn Pro Ile Gly Ala His Glu Ser Gly Leu Ile Gly Glu Gln
            180                 185                 190

ccc aac ggc gtt ccc aac aat ctt ttg ccc tat atc tgt caa gtg gct 624
Pro Asn Gly Val Pro Asn Asn Leu Leu Pro Tyr Ile Cys Gln Val Ala
        195                 200                 205

tcg ggc agg ctg ccg caa ctg tcg gta ttc ggc ggc gac tat ccg acc 672
Ser Gly Arg Leu Pro Gln Leu Ser Val Phe Gly Gly Asp Tyr Pro Thr
    210                 215                 220

ccc gac ggt acg gga atg cgc gac tac atc cat gtg atg gat ttg gca 720
Pro Asp Gly Thr Gly Met Arg Asp Tyr Ile His Val Met Asp Leu Ala
225                 230                 235                 240

gaa ggg cat atc gcg gca atg aag gcg aaa ggc ggc gtt gcc ggc gta 768
Glu Gly His Ile Ala Ala Met Lys Ala Lys Gly Gly Val Ala Gly Val
                245                 250                 255

cat ttg ttc aac ttg ggt tcg gga cgc gcc tat tcc gtt ttg gaa atc 816
His Leu Phe Asn Leu Gly Ser Gly Arg Ala Tyr Ser Val Leu Glu Ile
            260                 265                 270

atc cgc gcc ttt gag gcc gca tcc ggt ttg cac att cct tac cga atc 864
Ile Arg Ala Phe Glu Ala Ala Ser Gly Leu His Ile Pro Tyr Arg Ile
        275                 280                 285

caa ccc cgc cgc gcc ggc gac ttg gcg tgt tcc tat gcc gac ccg tcc 912
Gln Pro Arg Arg Ala Gly Asp Leu Ala Cys Ser Tyr Ala Asp Pro Ser
    290                 295                 300

cat acc aaa caa caa acc ggc tgg gaa acc aaa cgc ggc ttg cag caa 960
His Thr Lys Gln Gln Thr Gly Trp Glu Thr Lys Arg Gly Leu Gln Gln
305                 310                 315                 320

atg atg gaa gat tcg tgg cgt tgg gtc agc cgc aac ccc ggc aga tat 1008
Met Met Glu Asp Ser Trp Arg Trp Val Ser Arg Asn Pro Gly Arg Tyr
                325                 330                 335

ggg gat 1014
Gly Asp

<210> 5
<211> 36
<212> DNA
<213> Artificial Sequence

<223> Description of Artificial Sequence: Synthetic DNA

<400> 5
tataatcgat acaggtcatt ttttaggagg gtttac 36

<210> 6
<211> 36
<212> DNA
<213> Artificial Sequence
<223> Description of Artificial Sequence: Synthetic DNA

<400> 6
agaaggatcc cattcttatt ccgcactctt ataccc 36

<210> 7
<211> 30
<212> DNA
<213> Artificial Sequence
<223> Description of Artificial Sequence: Synthetic DNA

<400> 7
cccgatcgat tctgaaagga atgtttatga 30

<210> 8
<211> 28
<212> DNA

<213> Artificial Sequence
<223> Description of Artificial Sequence: Synthetic DNA

<400> 8
   ttgcggatcc tttgcattta atccccat                                    28


<210> 9
<211> 28
<212> DNA
<213> Artificial Sequence
<223> Description of Artificial Sequence: Synthetic DNA

<400> 9
aatactcgag atgctatttc aatcatac                                    28


<210> 10
<211> 26
<212> DNA
<213> Artificial Sequence
<223> Description of Artificial Sequence: Synthetic DNA

<400> 10
taaaggatcc ttaaaacaat gttaag                                    26


<210> 11
<211> 24
<212> DNA
<213> Artificial Sequence
<223> Description of Artificial Sequence: Synthetic DNA

<400> 11
caagaattct ctctcaccta ccaa                                    24


<210> 12
<211> 28
<212> DNA
<213> Artificial Sequence
<223> Description of Artificial Sequence: Synthetic DNA

<400> 12
aatctcgaga tcgataccct tttttacg                                          28

<210> 13
<211> 29
<212> DNA
<213> Artificial Sequence
<223> Description of Artificial Sequence: Synthetic DNA

<400> 13
aggaatcgat atgaaaataa gctttatta                                         29

<210> 14
<211> 36
<212> DNA
<213> Artificial Sequence
<223> Description of Artificial Sequence: Synthetic DNA

<400> 14
agttggatcc ataacagaaa gtttaggca                                         29

## Description

### Technical Field

The present invention relates to processes for producing UDP-N-acetylgalactosamine and an N-acetylgalactosamine-containing carbohydrate. Some N-acetylgalactosamine-containing carbohydrates are expressed specifically in cancer cells, etc. and thus are useful as anticancer vaccines, etc. for anticancer treatment. Further, as N-acetylgalactosamine-containing carbohydrates are present abundantly in the brain, they are expected to be applicable to the improvement of brain function. UDP-N-acetylgalactosamine is useful as a substrate for the synthesis of N-acetylgalactosamine-containing carbohydrates.

### Background Art

With regard to the production of UDP-N-acetylgalactosamine (hereinafter abbreviated as UDP-GalNAc), there are known processes for production utilizing a cell-free extract or a partially-purified enzyme derived from Bacillus subtilis [U.S. Patent No. 4,569,909; J. Biol. Chem., 234. 2801 (1959); Agr. Biol. Chem., 37, 1741 (1973); Appl. Environ. Microbiol., 41, 392 (1981); Microbiol. Immunol., 30, 1085 (1986); Agr. Biol. Chem., 49, 603 (1985)]. However, the productivity of UDP-GalNAc by the above methods is low, and UDP-N-acetylglucosamine 4-epimerase (EC 5.1.3.7, hereinafter abbreviated as UDP-GlcNAc 4-epimerase) derived from Bacillus subtilis or a gene encoding UDP-GlcNAc 4-epimerase has not been specified yet.

As regards UDP-GlcNAc 4-epimerase derived from a microorganism, there is a report that WbpP protein derived from Pseudomonas aeruginosa has UDP-GlcNAc 4-epimerase activity [J. Biol. Chem. , 275, 19060 (2000)], but no report on the production of UDP-GalNAc using the protein or DNA encoding the protein.

On the other hand, as for UDP-glucose 4-epimerase (EC 5.1.3.2), it is known that the enzymes derived from hamster and human also use UDP-GlcNAc as a substrate [Cell, 44, 749 (1986)], but there is no report that UDP-glucose 4-epimerase derived from Escherichia coli [Nucleic Acid Res., 14, 7705 (1986)] has UDP-GlcNAc 4-epimerase activity. Further, the above-mentioned WbpP protein derived from Pseudomonas aeruginosa has no homology to the known UDP-glucose 4-epimerases.

There are known proteins derived from Bacillus subtilis, Bacillus halodurans, Bacillus stearothermophilus, Haemophilus influenzae, Brucella abortus, Arabidopsis thaliana, Neisseria meningitidis, Neisseria gonorrhoeae, Erwinia amylovora, Yersinia enterocolitica, Campylobacter jejuni, Helicobacter pylori, Vibrio cholerae, Moraxella catarrhalis, Pisum sativurn, Salmonella, typhi, Salmonella typhimurium, Cyamopsis tetragonoloba, Pseudomonas aeruginosa, Lactococcus lactis, Aquifex aeolicus, Synechocystis sp. , Azospirium brasilense, Sinorhizobium meliloti, Rhizobium leguminosarum, Methanobacterium thermoautotrophicum, Corynebacterium glutamicum, Rhodococcus erythropolis, Rhodococcus equi, Streptomyces coelicolor, Streptomyces lividans, Deinococcus radiodurans, Bradyrhizobium japonicum, Thermotoga maritima, Klebsiella pneumoniae, Methanococcus jannaschii, Halobacterium sp. NRC-1, Pyrococcus horikoshii, Pyrococcus abyssi, Listeria monocytogenes, Clostridium perfringens, Deinococcus radiodurans. Archaeoglobus fulgidus, Staphylococcus carnosus and Streptococcus thermophilus which are regarded as UDP-glucose 4-epimerase (galE) because of their high homology to the amino acid sequence of a known UDP-glucose 4-epimerase. Also known are proteins derived from microorganisms such as Bacillus megaterium, Haemophilus ducreyi, Corynebacterium diphtheriae, Streptococcus pneumoniae and Streptococcus agalactiae which have high homology to the amino acid sequence of a known UDP-glucose 4-epimerase, but whose function has not been clarified yet. However, it is not known that these proteins have UDP-GlcNAc 4-epimerase activity and are useful for the production of UDP-GalNAc.

### Disclosure of the Invention

An object of the present invention is to provide processes for producing UDP-N-acetylgalactosamine and an N-acetylgalactosamine-containing carbohydrate.

The present inventors discovered that a protein which is regarded as UDP-glucose 4-epimerase (galE) because of its high homology to the amino acid sequence of a known UDP-glucose 4-epimerase or a protein which has high homology to the amino acid sequence of a known UDP-glucose 4-epimerase but whose function has not been clarified yet has UDP-N-acetylglucosamine 4-epimerase activity and that this enzyme is useful for the efficient production of UDP-N-acetylgalactosamine and an N-acetylgalactosamine-containing carbohydrate. The present invention has been completed based on the discovery.

The present invention relates to the following (1) to (35).
(1) A process for producing UDP-N-acetylgalactosamine (hereinafter abbreviated as UDP-GalNAc), which comprises:
   allowing an enzyme source and UDP-N-acetylglucosamine (hereinafter abbreviated as UDP-GlcNAc) to be present in an aqueous medium, said enzyme source being a culture of a transformant which produces a protein having UDP-N-acetylglucosamine 4-epimerase (hereinafter abbreviated as UDP-GlcNAc 4-epimerase) activity or a treated matter of the culture;
   allowing UDP-GalNAc to form and accumulate in the aqueous medium; and
   recovering UDP-GalNAc from the aqueous medium.
(2) A process for producing an N-acetylgalactosamine (hereinafter abbreviated as GalNAc)-containing carbohydrate, which comprises:
   allowing an enzyme source, an acceptor carbohydrate, GalNAc transferase and UDP-GlcNAc to be present in an aqueous medium, said enzyme source being a culture of a transformant which produces a protein having UDP-GlcNAc 4-epimerase activity or a treated matter of the culture;
   allowing the GalNAc-containing carbohydrate to form and accumulate in the aqueous medium; and
   recovering the GalNAc-containing carbohydrate from the aqueous medium.
(3) A process for producing UDP-GalNAc, which comprises:
   allowing enzyme sources, a precursor of uridine-5'-triphosphate (hereinafter abbreviated as UTP) and a sugar to be present in an aqueous medium, said enzyme sources being a culture of a microorganism having the ability to form UDP-GlcNAc from the precursor of UTP and the sugar or a treated matter of the culture, and
   a culture of a transformant which produces a protein having UDP-GlcNAc 4-epimerase activity or a treated matter of the culture;
   allowing UDP-GalNAc to form and accumulate in the aqueous medium; and
   recovering UDP-GalNAc from the aqueous medium.
(4) A process for producing a GalNAc-containing carbohydrate, which comprises:
   allowing enzyme sources, a precursor of UTP, a sugar and an acceptor carbohydrate to be present in an aqueous medium, said enzyme sources being a culture of a microorganism having the ability to form UDP-GlcNAc from the precursor of UTP and the sugar or a treated matter of the culture, GalNAc transferase, and a culture of a transformant which produces a protein having UDP-GlcNAc 4-epimerase activity or a treated matter of the culture;
   allowing the GalNAc-containing carbohydrate to form and accumulate in the aqueous medium; and
   recovering the GalNAc-containing carbohydrate from the aqueous medium.
(5) The process according to any of (1) to (4), wherein the treated matter of the culture is concentrated culture, dried culture, cells obtained by centrifuging the culture, a product obtained by subjecting the cells to drying, freeze-drying, treatment with a surfactant, ultrasonication, mechanical friction, treatment with a solvent, enzymatic treatment, protein fractionation or immobilization, or an enzyme preparation obtained by extracting the cells.
(6) The process according to (2) or (4), wherein the acceptor carbohydrate is a complex carbohydrate comprising an oligosaccharide having sialic acid, galactose, GalNAc, N-acetylglucosamine, fucose, glucuronic acid or iduronic acid at the nonreducing end.
(7) The process according to (6), wherein the oligosaccharide having sialic acid, galactose, GalNAc, N-acetylglucosamine, fucose, glucuronic acid or iduronic acid at the nonreducing end is lactose, N-acetyllactosamine, globotriose, sialyllactose, sialyl N-acetyllactosamine, Lewis X, Lewis a, sialyl Lewis X, sialyl Lewis a, chondroitin sulfate, dermatan sulfate, H type 1 (Fuc α1-2Gal β1-3GlcNAc) or H type 2 (Fuc *α1*-2Gal β1-4GlcNAc).
(8) The process according to (2) or (4), wherein the acceptor carbohydrate is lactose, N-acetyllactosamine, globotriose, sialyllactose, sialyl N-acetyllactosamine, Lewis X, Lewis a, sialyl Lewis X, sialyl Lewis a, chondroitin sulfate, dermatan sulfate, H type 1 (Fuc α 1-2Gal β 1-3GlcNAc) or H type 2 (Fuc α 1-2Gal β1-4GlcNAc) .
(9) The process according to (3) or (4), wherein the precursor is orotic acid, orotidine, uracil, uridine or uridine-5'-monophosphate.
(10) The process according to (3) or (4), wherein the sugar is glucosamine or N-acetylglucosamine.
(11) The process according to (3) or (4), wherein the microorganism having the ability to form UDP-GlcNAc from the precursor of UTP and the sugar is one or more microorganisms selected from the group consisting of microorganisms belonging to the genera Escherichia, Corynebacterium and Saccharomyces.
(12) The process according to (11), wherein the microorganism belonging to the genus Escherichia is Escherichia coli.
(13) The process according to (11), wherein the microorganism belonging to the genus Corynebacterium is Corynebacterium ammoniagenes.
(14) The process according to (11), wherein the microorganism belonging to the genus Saccharomyces is Saccharomyces cerevisiae.
(15) The process according to any of (1) to (4), wherein the transformant is a transformant obtained by introducing a recombinant DNA into a microorganism.
(16) The process according to (15), wherein the microorganism is selected from the group consisting of microorganisms belonging to the genera Escherichia, Corynebacterium and Saccharomyces.
(17) The process according to (16), wherein the microorganism belonging to the genus Escherichia is Escherichia coli.
(18) The process according to (16), wherein the microorganism belonging to the genus Corynebacterium is Corynebacterium glutamicum.
(19) The process according to (16), wherein the microorganism belonging to the genus Saccharomyces is Saccharomyces cerevisiae.
(20) The process according to any of (1) to (4), wherein the protein having UDP-GlcNAc 4-epimerase activity is a protein having UDP-GlcNAc 4-epimerase activity which is derived from a microorganism belonging to the genus Bacillus or Neisseria.
(21) The process according to (20), wherein the microorganism belonging to the genus Bacillus is selected from the group consisting of Bacillus subtilis, Bacillus megaterium and Bacillus stearothermophilus.
(22) The process according to (20), wherein the microorganism belonging to the genus Neisseria is Neisseria gonorrhoeae or Neisseria meningitidis.
(23) The process according to any of (1) to (4) and (20) to (22), wherein the protein having UDP-GlcNAc 4-epimerase activity is a protein having the amino acid sequence shown in SEQ ID NO: 1 or 2.
(24) The process according to any of (1) to (4) and (20) to (22), wherein the protein having UDP-GlcNAc 4-epimerase activity is a protein consisting of an amino acid sequence wherein one or more amino acid residues are deleted, substituted, inserted or added in the amino acid sequence shown in SEQ ID NO: 1 or 2 and having UDP-GlcNAc 4-epimerase activity.
(25) The process according to any of (1) to (4) and (20) to (22), wherein the protein having UDP-GlcNAc 4-epimerase activity is a protein consisting of an amino acid sequence having 50% or more homology to the amino acid sequence shown in SEQ ID NO: 1 or 2.
(26) The process according to (15), wherein the recombinant DNA comprises DNA encoding a protein having UDP-GlcNAc 4-epimerase activity.
(27) The process according to (15), wherein the recombinant DNA comprises DNA encoding UDP-glucose 4-epimerase (hereinafter abbreviated as galE protein) having UDP-GlcNAc 4-epimerase activity.
(28) The process according to (27), wherein the galE protein is derived from a microorganism belonging to the genus Bacillus or Neisseria.
(29) The process according to (28), wherein the microorganism belonging to the genus Bacillus is selected from the group consisting of Bacillus subtilis, Bacillus megaterium and Bacillus stearothermophilus.
(30) The process according to (28), wherein the microorganism belonging to the genus Neisseria is Neisseria gonorrhoeae or Neisseria meningitidis.
(31) The process according to (15), wherein the recombinant DNA comprises DNA encoding a protein having the amino acid sequence shown in SEQ ID NO: 1 or 2.
(32) The process according to (15), wherein the recombinant DNA comprises DNA encoding a protein consisting of an amino acid sequence wherein one or more amino acid residues are deleted, substituted, inserted or added in the amino acid sequence shown in SEQ ID NO: 1 or 2 and having UDP-GlcNAc 4-epimerase activity.
(33) The process according to (15), wherein the recombinant DNA comprises DNA encoding a protein consisting of an amino acid sequence which has 50% or more homology to the amino acid sequence shown in SEQ ID NO: 1 or 2 and having UDP-GlcNAc 4-epimerase activity.
(34) The process according to (15), wherein the recombinant DNA comprises DNA having the nucleotide sequence shown in SEQ ID NO: 3 or 4.
(35) The process according to (15), wherein the recombinant DNA comprises DNA which hybridizes with DNA consisting of the nucleotide sequence shown in SEQ ID NO: 3 or 4 under stringent conditions and which encodes a protein having UDP-GlcNAc 4-epimerase activity.

Any proteins having UDP-GlcNAc 4-epimerase activity can be used in the process of the present invention. Suitable examples are the following (1) to (6).
(1) A galE protein having UDP-GlcNAc 4-epimerase activity
(2) A protein which is supposed to have the function of galE protein because of its high homology to the amino acid sequence of galE protein and which has UDP-GlcNAc 4-epimerase activity
(3) A protein having an amino acid sequence which has 50% or more homology to the amino acid sequence of galE protein and having UDP-GlcNAc 4-epimerase activity
(4) A protein having the amino acid sequence shown in SEQ ID NO: 1 or 2
(5) A protein consisting of an amino acid sequence wherein one or more amino acid residues are deleted, substituted, inserted or added in the amino acid sequence shown in SEQ ID NO: 1 or 2 and having UDP-GlcNAc 4-epimerase activity
(6) A protein consisting of an amino acid sequence which has 50% or more homology to the amino acid sequence shown in SEQ ID NO: 1 or 2 and having UDP-GlcNAc 4-epimerase activity

Examples of the galE protein having UDP-GlcNAc 4-epimerase activity are those derived from Bacillus subtilis (GenBank accession No. X99339) and Neisseria gonorrhoeae (GenGank accession No. Z215508).

Examples of the proteins regarded as galE protein because of their high homology to the amino acid sequence of a known galE protein are those derived from Bacillus halodurans (GenBank accession No. AP001510), Haemophilus influenzae (GenBank accession No. X57315), Brucella abortus (GenBank accession No. U78089), Arabidopsis thaliana (GenBank accession No. AL078468), Neisseria meningitidis (GenBank accession No. AF083467), Erwinia amylovora (GenBank accession No. X76172), Yersinia enterocolitica (GenBank accession No. Z47767), Campylobacter jejuni (GenBank accession No. CJ11168X4), Helicobacter pylori (GenBank accession No. AF016844), Vibrio cholerae (GenBank accession No. AE004405, Moraxella catarrhalis (GenBank accession No. AF248584), Pisum sativum (GenBank accession No. U31544), Salmonella typhi (GenBank accession No. X83927), Salmonella typhimurium (GenBank accession No. M33681), Cyamopsis tetragonoloba (GenBank accession No. AJ005081), Pseudomonas aeruginosa (GenBank accession No. AE004568), Lactococcus lactis (GenBank accession No. AJ011653), Aquifex aeolicus (GenBank accession No. AE000721), Synechocystis sp. (GenBank accession No. D90910), Azospirium brasilense (GenBank accession No. U09349), Sinorhizobium meliloti (GenBank accession No. X58126), Rhizobiurn leguminosarum (GenBank accession No. X96507), Methanobacterium thermoautotrophicum (GenBank accession No. AE000844), Corynebacterium glutamicum (GenBank accession No. Z49823), Rhodococcus erythropolis (GenBank accession No. AF221951), Rhodococcus equi (GenBank accession No. AF277002), Streptomyces coelicolor (GenBank accession No. AL359989), Streptomyces lividans (GenBank accession No. M18953), Deinococcus radiodurans (GenBank accession No. AE002053), Bradyrhizobium japonicum (GenBank accession No. AF253311), Thermotoga maritima (GenBank accession No. AE001727), Klebsiella pneumoniae (GenBank accession No. M94964), Methanococcus jannaschii (GenBank accession No. U67477), Halobacterium sp. NRC-1 (GenBank accession No. AE004975), Pyrococcus horikoshii (GenBank accession No. AP000007), Pyrococcus abyssi (GenBank accession No. AJ248284), Listeria monocytogenes (GenBank accession No. AF0099622), Clostridium perfringens (GenBank accession No. X86505), Deinococcus radiodurans (GenBank accession No. AE001927), Archaeoglobus fulgidus (GenBank accession No. AE001079), Staphylococcus carnosus (GenBank accession No. AF109295) and Streptococcus thermophilus (GenBank accession No. M38175).

Examples of the proteins having an amino acid sequence which has 50% or more homology to the amino acid sequence of galE protein and having UDP-GlcNAc 4-epimerase activity are that derived from Corynebacterium diphtheriae (GenBank accession No. M80338) and those derived from microorganisms such as Bacillus megaterium, Bacillus stearothermophilus, Haemophilus ducreyi, Streptococcus pneumoniae and Streptococcus agalactiae.

Examples of the proteins having an amino acid sequence which has 50% or more homology to the amino acid sequence of galE protein are those consisting of an amino acid sequence which has at least 50% or more, preferably 60% or more, more preferably 80% or more, further preferably 95% or more homology to the amino acid sequence of galE protein.

The homology among amino acid sequences and nucleotide sequences can be determined using algorithm BLAST by Karlin and Altschul [Pro. Natl. Acad. Sci. USA, 90, 5873 (1993)] and FASTA [Methods Enzymol., 183, 63 (1990)]. On the basis of the algorithm BLAST, programs such as BLASTN and BLASTX have been developed [J. Mol. Biol., 215, 403 (1990)]. When a nucleotide sequence is analyzed by BLASTN on the basis of BLAST, the parameters, for instance, are as follows: score=100 and wordlength=12. When an amino acid sequence is analyzed by BLASTX on the basis of BLAST, the parameters, for instance, are as follows: score=50 and wordlength=3. When BLAST and Gapped BLAST programs are used, default parameters of each program are used. The specific techniques for these analyses are known (http://www.ncbi.nlm.nih.gov.).

More specific examples of the proteins having UDP-GlcNAc 4-epimerase activity useful in the process of the present invention are proteins having UDP-GlcNAc 4-epimerase activity derived from microorganisms belonging to the genus Bacillus such as Bacillus subtilis, Bacillus megaterium and Bacillus stearothermophilus, and those belonging to the genus Neisseria such as Neisseria gonorrhoeae and Neisseria meningitidis.

The protein consisting of an amino acid sequence wherein one or more amino acid residues are deleted, substituted or added and having UDP-GlcNAc 4-epimerase activity can be obtained, for example, by introducing a mutation into DNA encoding a protein having the amino acid sequence shown in SEQ ID NO: 1 or 2 by site-directed mutagenesis described in Molecular Cloning, A Laboratory Manual, Second Edition (1989) (hereinafter abbreviated as Molecular Cloning, Second Edition); Current Protocols in Molecular Biology, John Wiley & Sons (1987-1997) (hereinafter abbreviated as Current Protocols in Molecular Biology); Nucleic Acids Res., 10, 6487 (1982); Proc. Natl. Acad. Sci. USA, 79, 6409 (1982); Gene, 34, 315 (1985); Nucleic Acids Res., 13, 4431 (1985); Proc. Natl. Acad. Sci. USA, 82, 488 (1985), etc.

The number of amino acid residues which are deleted, substituted, inserted or added is not specifically limited, but is within the range where deletion, substitution, insertion or addition is possible by known methods such as the above site-directed mutagenesis. The suitable number is 1 to dozens, preferably 1 to 20, more preferably 1 to 10, further preferably 1 to 5.

The expression "one or more amino acid residues are deleted, substituted, inserted or added in the amino acid sequence of a polypeptide having UDP-GlcNAc 4-epimerase activity" means that the amino acid sequence contains deletion, substitution, insertion or addition of a single or plural amino acid residues at a single or plural arbitrary positions therein. Deletion, substitution, insertion and addition may be simultaneously contained in one sequence, and amino acid residues to be substituted, inserted or added may be either natural or not. Examples of the natural amino acid residues are L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine and L-cysteine.

The following are examples of the amino acid residues capable of mutual substitution. The amino acid residues in the same group can be mutually substituted.
Group A: leucine, isoleucine, norleucine, valine, norvaline, alanine, 2-aminobutanoic acid, methionine, O-methylserine, t-butylglycine, t-butylalanine, cyclohexylalanine
Group B: aspartic acid, glutamic acid, isoaspartic acid, isoglutamic acid, 2-aminoadipic acid, 2-aminosuberic acid
Group C: asparagine, glutamine
Group D: lysine, arginine, ornithine, 2,4-diaminobutanoic acid, 2,3-diaminopropionic acid
Group E: proline, 3-hydroxyproline, 4-hydroxyproline
Group F: serine, threonine, homoserine
Group G: phenylalanine, tyrosine

In order that the protein to be used in the process of the present invention may have UDP-GlcNAc 4-epimerase activity, it is desirable that the homology of its amino acid sequence to the amino acid sequence shown in SEQ ID NO: 1 or 2 is at least 50% or more, preferably 60% or more, more preferably 80% or more, further preferably 95% or more, when calculated using BLAST, FASTA, etc. under the above conditions.

Examples of the DNAs encoding the proteins having UDP-GlcNAc 4-epimerase activity useful in the process of the present invention are those encoding the above proteins (1) to (6).

Specific examples include DNA having the nucleotide sequence shown in SEQ ID NO: 3 or 4, and DNA which hybridizes with this DNA under stringent conditions and which encodes a protein having UDP-GlcNAc 4-epimerase activity.

The above DNA capable of hybridization under stringent conditions refers to DNA which is obtained by colony hybridization, plaque hybridization, Southern blot hybridization, or the like using a part or the whole of DNA encoding the protein according to any of the above (1) to (6) as a probe. A specific example of such DNA is DNA which can be identified by performing hybridization at 65°C in the presence of 0.7 to 1.0 mol/l sodium chloride using a filter with colony- or plaque-derived DNA immobilized thereon, and then washing the filter at 65°C with a 0.1 to 2-fold conc. SSC solution (1-fold conc. SSC solution: 150 mmol/l sodium chloride and 15 mmol/l sodium citrate). Hybridization can be carried out according to the methods described in Molecular Cloning, Second Edition; Current Protocols in Molecular Biology; DNA Cloning 1: Core Techniques, A Practical Approach, Second Edition, Oxford University (1995), etc. Specifically, the hybridizable DNA includes DNA having at least 60% or more homology, preferably 80% or more homology, further preferably 95% or more homology to the nucleotide sequence shown in SEQ ID NO: 3 or 4 as calculated by use of BLAST or FASTA under the above conditions.

The transformant which produces a protein having UDP-GlcNAc 4-epimerase activity used in the process of the present invention can be obtained, for example, by preparing a recombinant DNA by ligating the DNA encoding the protein prepared by the above-described method to a vector DNA according to the method described in Molecular Cloning, Second Edition, and then transforming a host cell using the recombinant DNA according to the method described in Molecular Cloning, Second Edition.

Preparation of the transformant which expresses a protein having UDP-GlcNAc 4-epimerase activity used in the process of the present invention is described below.

### (1) Preparation of DNA Encoding a Protein Having UDP-GlcNAc 4-Epimerase Activity

The DNA encoding a protein having UDP-GlcNAc 4-epimerase activity can be prepared from DNA derived from any organism having the enzyme activity. Examples of the suitable DNAs are those derived from microorganisms, preferably those belonging to the genus Bacillus or Neisseria, more preferably Bacillus subtilis MI112 (ATCC 33712) and Neisseria gonorrhoeae (ATCC 33084).

Microorganisms belonging to the genera Bacillus and Neisseria can be cultured by a known method. After the culturing, the chromosomal DNA of the microorganism is isolated and purified by a known method (for example, Current Protocols in Molecular Biology).

A fragment comprising the DNA encoding a protein having UDP-GlcNAc 4-epimerase activity can be obtained by PCR [PCR Protocols, Hamana Press (1993)] using a set of primers prepared on the basis of the nucleotide sequence of a known DNA encoding a protein supposed to have UDP-glucose 4-epimerase (EC 5.1.3.2) activity and, as a template, the chromosomal DNA. Examples of the suitable nucleotide sequences are that derived from Bacillus subtilis shown in SEQ ID NO: 1 and that derived from Neisseria gonorrhoeae shown in SEQ ID NO: 2.

The desired DNA can also be obtained by hybridization using a synthetic DNA designed based on a known nucleotide sequence as a probe.

It is also possible to artificially synthesize the DNA encoding a protein having UDP-GlcNAc 4-epimerase activity by PCR using a synthetic DNA according to a conventional method [PCR Protocols, Hamana Press (1993)].

### (2) Cloning of DNA Encoding a Protein Having UDP-GlcNAc 4-Epimerase Activity and Confirmation of the Nucleotide Sequence

The DNA to be used in the process of the present invention prepared in the above (1), as such or after cleavage with appropriate restriction enzymes, is ligated to a vector by a conventional method.

As the vector to which the DNA is ligated, any phage vectors, plasmid vectors, etc. that are capable of autonomous replication in Escherichia coli K12 can be employed. Examples of suitable vectors are ZAP Express [STRATAGENE; Strategies, 5, 58 (1992)], pBluescript II SK(+) [STRATAGENE; Nucleic Acids Res., 17. 9494 (1989)], λzap II (STRATAGENE), λgt10, λgt11 [DNA Cloning, A Practical Approach, 1, 49 (1985)], λTriplEx (Clontech) , λExCell (Amersham Pharmacia Biotech) and pUC18 [Gene, 33, 103 (1985)].

As the Escherichia coli used as host cells for a recombinant DNA obtained by ligating the DNA used in the process of the present invention obtained in (1) to the vector, any microorganism belonging to Escherichia coli can be employed. Examples of suitable microorganisms are Escherichia coli XL1-Blue MRF' [STRATAGENE; Strategies, 5, 81 (1992)], Escherichia coli C600 [Genetics, 39, 440 (1954)], Escherichia coli Y1088 [Science, 222, 778 (1983)], Escherichia coli Y1090 [Science, 222, 778 (1983)], Escherichia coli NM522 [J. Mol. Biol., 166. 1 (1983)], Escherichia coli K802 [J. Mol. Biol., 16, 118 (1966)] and Escherichia coli JM105 [Gene, 38, 275 (1985)].

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

The nucleotide sequence of the DNA of the present invention contained in the recombinant DNA can be determined after extracting the recombinant DNA from the transformant obtained in the above manner. Determination of the nucleotide sequence can be carried out by a conventional sequencing method such as the dideoxy method [Proc. Natl. Acad. Sci. USA, 74, 5463 (1977)] or by using a nucleotide sequencer such as 373A DNA Sequencer (Perkin-Elmer Corp.).

Examples of the transformants carrying the recombinant DNA obtained in the above manner are Escherichia coli NM522/pGT73 carrying a plasmid DNA having the nucleotide sequence shown in SEQ ID NO: 3 and Escherichia coli NM522/pGT24 carrying a plasmid DNA having the nucleotide sequence shown in SEQ ID NO: 4.

### (3) Preparation of a Transformant Which Produces a Protein Having UDP-GlcNAc 4-Epimerase Activity

Preparation of the transformant which produces a protein having UDP-GlcNAc 4-epimerase activity can be carried out, for example, in the following manner by use of the methods described in Molecular Cloning, Second Edition and Current Protocols in Molecular Biology.

That is, on the basis of the DNA obtained above, a DNA fragment of an appropriate length comprising a region encoding the protein is prepared according to need, and the DNA fragment is inserted downstream of a promoter in an appropriate expression vector to prepare a recombinant DNA. Then, the recombinant DNA is introduced into a host cell suited for the expression vector to prepare a transformant.

As the host cell, any bacterial cells, yeast cells, etc. that are capable of expressing the desired gene can be used.

The expression vectors that can be employed are those capable of autonomous replication or integration into the chromosomal DNA in the above host cells and comprising a promoter at a position appropriate for the transcription of the DNA encoding the protein of the present invention.

When a procaryote such as a bacterium is used as the host cell, it is preferred that the recombinant DNA comprising the DNA encoding a protein having UDP-GlcNAc 4-epimerase activity is a vector which is capable of autonomous replication in the procaryote and which comprises a promoter, a ribosome binding sequence, the DNA of the present invention and a transcription termination sequence. The vector may further comprise a gene regulating the promoter.

Examples of suitable expression vectors are pHelix1 (Roche Diagnostics), pKK233-2 (Amersham Pharmacia Biotech), pSE280 (Invitrogen), pGEMEX-1 (Promega), pQE-8 (QIAGEN), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82. 4306 (1985)], pBluescript II SK(-) (STRATAGENE), pTrs30 [prepared from Escherichia coli JM109/pTrS30 (FERM BP-5407)], pTrs32 [prepared from Escherichia coli JM109/pTrS32 (FERM BP-5408)], pPAC31 (WO98/12343), pGHA2 [prepared from Escherichia coli IGHA2 (FERM B-400); Japanese Published Unexamined Patent Application No. 221091/85], pGKA2 [prepared from Escherichia coli IGKA2 (FERM BP-6798); Japanese Published Unexamined Patent Application No. 221091/85], pTerm2 (US4686191, US4939094 and US5160735), pSupex, pUB110, pTP5, pC194, pEG400 [J. Bacteriol., 172. 2392 (1990)], pGEX (Amersham Pharmacia Biotech) and pET system (Novagen).

As the promoter, any promoters capable of functioning in host cells can be used. For example, promoters derived from Escherichia, coli or phage, such as trp promoter (Pₜᵣₚ), lac promoter, P_{L} promoter, P_{R} promoter and T7 promoter can be used. Artificially designed and modified promoters such as a promoter in which two Pₜᵣₚs are combined in tandem (Pₜᵣₚ x 2), tac promoter, lacT7 promoter and letI promoter, etc. can also be used.

It is preferred to use a plasmid in which the distance between the Shine-Dalgarno sequence (ribosome binding sequence) and the initiation codon is adjusted to an appropriate length (e.g., 6 to 18 bases).

In the recombinant DNA of the present invention, the transcription termination sequence is not essential for the expression of the DNA of the present invention, but it is preferred to place the transcription termination sequence immediately downstream of the structural gene.

Examples of suitable host cells are microorganisms belonging to the genera Escherichia, Serratia, Bacillus, Brevibacterium, Corynebacterium, Microbacterium and Pseudomonas. Specific examples are Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli GI698, Escherichia coli TB1, Serratia ficaria, Serratia fonticola, Serratia liquefaciens, Serratia marcescens, Bacillus subtilis, Bacillus megaterium, Bacillus amyloliquefaciens, Corynebacterium ammoniagenes, Brevibacterium immariophilum ATCC 14068, Brevibacterium saccharolyticurn ATCC 14066, Brevibacterium flavum ATCC 14067, Brevibacterium lactofermentum ATCC 13869, corynebacterium glutamicum ATCC 13032, Corynebacterium glutamicum ATCC 13869, Corynebacterium acetoacidophilum ATCC 13870, Microbacterium ammoniaphilum ATCC 15354, Pseudomonas putida and Pseudomonas sp. D-0110.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and the methods described in Gene, 17, 107 (1982) and Mol. Gen. Genet., 168, 111 (1979).

When yeast is used as the host cell, YEP13 (ATCC 37115), YEp24 (ATCC 37051), YCpS (ATCC 37419), pHS19, pHS15, etc. can be used as the expression vector.

As the promoter, any promoters capable of functioning in yeast can be used. Suitable promoters include the promoter of a glycolytic gene such as hexose kinase, PHO5 promoter, PGK promoter, GAP promoter, ADH promoter, gal 1 promoter, gal 10 promoter, heat shock polypeptide promoter, MFα1 promoter and CUP 1 promoter.

Examples of suitable host cells are microorganisms belonging to the genera Saccharomyces, Schizosaccharomyces, Kluyveromyces, Trichosporon, Schwanniomyces, Pichia and Candida, specifically, Saccharomyces cerevisiae, Schizosaccharomyces pombe, Kluyveromyces lactis, Trichosporon pullulans, Schwanniomyces alluvius and Candida utilis.

Introduction of the recombinant DNA can be carried out by any of the methods for introducing DNA into yeast, for example, electroporation [Methods Enzymol., 194, 182 (1990)], the spheroplast method [Proc. Natl. Acad. Sci. USA, 75, 1929 (1978)], the lithium acetate method [J. Bacteriol., 153, 163 (1983)] and the method described in Proc. Natl. Acad. Sci. USA, 75, 1929 (1978).

Culturing of the above-prepared transformant used for the preparation of UDP-GalNAc and a GalNAc-containing carbohydrate can be carried out by conventional methods for culturing the host.

When the transformant used in the process of the present invention is prepared by using a procaryote such as Escherichia coli or a eucaryote such as yeast as the host, any of natural media and synthetic media can be used as a medium for culturing the transformant insofar as it is a medium suitable for efficient culturing of the transformant which contains carbon sources, nitrogen sources, inorganic salts, etc. which can be assimilated by the transformant.

As the carbon sources, any carbon sources that can be assimilated by the transformant can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

As the nitrogen sources, ammonia, ammonium salts of organic or inorganic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented microbial cells and digested products thereof.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is carried out under aerobic conditions, for example, by shaking culture or submerged spinner culture under aeration, at 15 to 40°C usually for 16 hours to 7 days. The pH is preferably maintained at 3.0 to 9.0 during the culturing. The pH adjustment is carried out by using an organic or inorganic acid, an alkali solution, urea, calcium carbonate, ammonia, etc.

If necessary, antibiotics such as ampicillin, tetracycline and chloramphenicol may be added to the medium during the culturing.

When a microorganism transformed with a recombinant DNA comprising an inducible promoter is cultured, an inducer may be added to the medium, if necessary. For example, in the case of a microorganism transformed with a recombinant DNA comprising lac promoter, isopropyl- β -D-thiogalactopyranoside or the like may be added to the medium; and in the case of a microorganism transformed with a recombinant DNA comprising trp promoter, indoleacrylic acid or the like may be added.

The thus obtained culture of the transformant and various treated matters of the culture can be used as an enzyme source for the production of UDP-GalNAc or a GalNAc-containing carbohydrate in an aqueous medium.

The treated matters of the culture include concentrated culture, dried culture, cells obtained by centrifuging the culture, products obtained by treating the cells by various means such as drying, freeze-drying, treatment with a surfactant, ultrasonication, mechanical friction, treatment with a solvent, enzymatic treatment, protein fractionation and immobilization, an enzyme preparation obtained by extracting the cells, etc.

In the formation of UDP-GalNAc or a GalNAc-containing carbohydrate, the enzyme source is used at a concentration of 0.1 mU/l to 10,000 U/l, preferably 1 mU/l to 1,000 U/l, one unit (U) being defined as the activity which forms 1 µ mol of UDP-GalNAc or a GalNAc-containing carbohydrate at 37°C in one minute.

Aqueous media useful in the formation of UDP-GalNAc or a GalNAc-containing carbohydrate include water, buffers such as phosphate buffer, carbonate buffer, acetate buffer, borate buffer, citrate buffer and Tris buffer, alcohols such as methanol and ethanol, esters such as ethyl acetate, ketones such as acetone, amides such as acetamide, etc. The culture of the microorganism used as the enzyme source can be used also as the aqueous medium.

If necessary, a surfactant or an organic solvent may be added in the formation of UDP-GalNAc or a GalNAc-containing carbohydrate. Any surfactant that promotes the formation of a GalNAc-containing carbohydrate can be used. Suitable surfactants include nonionic surfactants such as polyoxyethylene octadecylamine (e.g., Nymeen S-215, NOF Corporation), cationic surfactants such as cetyltrimethylammonium bromide and alkyldimethylbenzylammonium chloride (e.g., Cation F2-40E, NOF Corporation), anionic surfactants such as lauroyl sarcosinate, and tertiary amines such as alkyldimethylamine (e.g., Tertiary Amine FB, NOF Corporation), which may be used alone or in combination.

The surfactant is usually used at a concentration of 0.1 to 50 g/l. As the organic solvent, xylene, toluene, aliphatic alcohols, acetone, ethyl acetate, etc. may be used usually at a concentration of 0.1 to 50 ml/l.

UDP-GlcNAc useful in the formation of UDP-GalNAc and a GalNAc-containing carbohydrate includes commercially available ones, reaction solutions formed utilizing the activity of a microorganism or the like, and those purified from the reaction solutions. Examples of suitable microorganisms are those belonging to the genera Escherichia, Corynebacterium and Saccharomyces, specifically, Escherichia coli, Corynebacterium ammoniagenes and Saccharomyces cerevisiae. These microorganisms may be used alone or in combination. The sugar nucleotide substrate is used at a concentration of 0.1 to 500 mmol/l.

UDP-GalNAc can be produced by allowing the above enzyme source, a culture of a microorganism having the ability to form UDP-GlcNAc from a precursor of UTP and a sugar or a treated matter of the culture as an additional enzyme source, the precursor of UTP and the sugar to be present in an aqueous medium, allowing UDP-GalNAc to form and accumulate in the aqueous medium, and recovering UDP-GalNAc from the aqueous medium.

Examples of the precursor of UTP used in the present process include orotic acid, orotidine, uridine, uracil and uridine-5'-monophosphate. The precursor may be a purified product, a salt of the precursor, or a culture containing the precursor produced by a microorganism or a partially-purified product obtained from the culture insofar as contaminants therein do not inhibit the reaction. The precursor is used at a concentration of 0.1 mmol/l to 1.0 mol/l, preferably 0.01 to 0.5 mol/l.

Examples of the sugar used in the present process include glucosamine and N-acetylglucosamine. The sugar may be a purified product or any product containing the sugar insofar as contaminants therein do not inhibit the reaction. The sugar can be added at a time at the start of the reaction, or in portions or continuously during the reaction and is used at a concentration of 0.1 mmol/l to 2.0 mol/l.

A GalNAc-containing carbohydrate can be produced by allowing the above enzyme source, a culture of a microorganism having the ability to form UDP-GlcNAc from a precursor of UTP and a sugar or a treated matter of the culture and GalNAc transferase as additional enzyme sources, the precursor of UTP, the sugar and an acceptor carbohydrate to be present in an aqueous medium, allowing the GalNAc-containing carbohydrate to form and accumulate in the aqueous medium, and recovering the GalNAc-containing carbohydrate from the aqueous medium.

The acceptor carbohydrate used in the formation of a GalNAc-containing carbohydrate may be any acceptor carbohydrate that works as a substrate for GalNAc transferase. An example of a suitable acceptor carbohydrate is an acceptor carbohydrate which contains an oligosaccharide having sialic acid, galactose, GalNAc, N-acetylglucosamine, fucose, glucuronic acid or iduronic acid at the nonreducing end. Examples of the oligosaccharides having sialic acid, galactose, GalNAc, N-acetylglucosamine, fucose, glucuronic acid or iduronic acid at the nonreducing end are lactose, N-acetyllactosamine, globotriose, sialyllactose, sialyl N-acetyllactosamine, Lewis X, Lewis a, sialyl Lewis X, sialyl Lewis a, chondroitin sulfate, dermatan sulfate, H type 1 (Fuc α1-2Gal β1-3GlcNAc) and H type 2 (Fuc α 1-2Gal β 1-4GlcNAc). Preferred acceptor carbohydrates are lactose, N-acetyllactosamine, globotriose, sialyllactose, sialyl N-acetyllactosamine, Lewis X, Lewis a, sialyl Lewis X, sialyl Lewis a, chondroitin sulfate, dermatan sulfate, H type 1 (Fuc α 1-2Gal β 1-3GlcNAc) and H type 2 (Fuc α1-2Gal β 1-4GlcNAc).

The acceptor carbohydrate is used at a concentration of 0.1 to 500 mmol/l.

If necessary, an inorganic salt such as MnCl₂, β-mercaptoethanol, and the like may be added in the above reaction for forming UDP-GalNAc and a GalNAc-containing carbohydrate.

The reaction for the formation of UDP-GalNAc and a GalNAc-containing carbohydrate is carried out in an aqueous medium at pH 5 to 10, preferably pH 6 to 8, at 20 to 50°C for 1 to 96 hours.

Determination of UDP-GalNAc and the GalNAc-containing carbohydrate formed in the aqueous medium can be carried out according to known methods [Microbiol. Immunol., 30, 1085 (1986); Kagaku to Kogyo (Chemistry and Chemical Industry), 43, 953 (1990)].

Recovery of UDP-GalNAc and the GalNAc-containing carbohydrate formed in the reaction mixture can be carried out by ordinary methods using active carbon, ion-exchange resins, etc. For example, UDP-GalNAc can be recovered by the method described in Agr. Biol. Chem., 37, 1741 (1973), and the GalNAc-containing carbohydrate by the method described in J. Org. Chem., 47, 5416 (1982).

### Brief Description of the Drawings

Fig. 1 shows the steps for constructing plasmid pGT73 expressing a gene encoding a protein having UDP-GlcNAc 4-epimerase activity derived from Bacillus subtilis.
Fig. 2 shows the steps for constructing plasmid pGT24 expressing a gene encoding a protein having UDP-GlcNAc 4-epimerase activity derived from Neisseria gonorrhoeae.
Fig. 3 shows the steps for constructing plasmid pGT87 expressing the β1,4-N-acetylgalactosaminyltransferase gene derived from Campylobacter jejuni
Fig. 4 shows the steps for constructing plasmid pCJ2 expressing the α1,4-N-acetylgalactosaminyltransferase gene derived from Campylobacter jejuni.

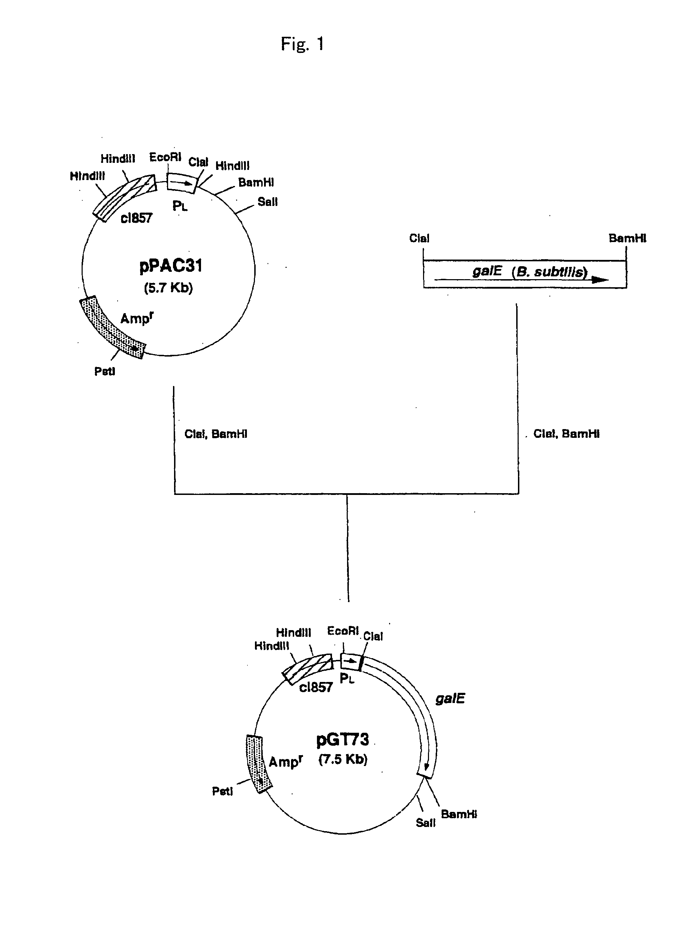

The symbols in Figs. 1 to 4 refer to the following.
P_{L}: P_{L} promoter
cI857: cI857 repressor gene
Amp^{r}: ampicillin resistance gene
galE (B. subtilis): gene encoding a protein having UDP-GlcNAc 4-epimerase activity derived from Bacillus subtilis
galE (N. gonorrhoeae): gene encoding a protein having UDP-GlcNAc 4-epimerase activity derived from Neisseria gonorrhoeae
beta 1,4-GalNAc transferase: β1,4-N-acetylgalactosaminyltransferase gene derived from Campylobacter jejuni
alpha 1,4-GalNAc transferase: α1, 4-N-acetylgalactosaminyltransferase gene derived from Campylobacter jejuni

### Best Modes for Carrying Out the Invention

Examples of the present invention are shown below. These examples are not to be construed as limiting the scope of the invention.

### Example 1

### Construction of a Transformant Which Produces UDP-glucose 4-Epimerase Derived from Bacillus subtilis

Bacillus subtilis MI112 (ATCC 33712) was inoculated into 20 ml of LB medium (10 g/l Bacto-tryptone, 10 g/l yeast extract and 5 g/l sodium chloride) in a 300-ml flask and cultured at 37°C for 16 hours. After the culturing, the chromosomal DNA of the microorganism was prepared according to the method described in Current Protocols in Molecular Biology.

On the basis of the nucleotide sequence of the galE gene supposed to encode a protein having UDP-glucose 4-epimerase activity derived from Bacillus subtilis [Microbiology, 142, 3113 (1996)], DNAs having the nucleotide sequences shown in SEQ ID NOS: 5 and 6 were synthesized using a DNA synthesizer (Model 8905, PerSeptive Biosystems). PCR was carried out in the following manner using the synthesized DNAs as a set of primers and the chromosomal DNA of Bacillus subtilis MI112 as a template. That is, PCR was carried out by 30 cycles, one cycle consisting of reaction at 94°C for one minute, reaction at 37°C for 2 minutes and reaction at 72°C for 3 minutes, using 40 µl of a reaction mixture comprising 0.1 µg of the chromosomal DNA, 0.5 µ mol/l each of the primers, 2.5 units of Pfu DNA polymerase (STRATAGENE), 4 µl of buffer for Pfu DNA polymerase (10 x) (STRATAGENE) and 200 µmol/l each of deoxyNTPs, whereby a ca. 1.0 kb PCR product was obtained.

One-tenth of the resulting reaction mixture was subjected to agarose gel electrophoresis to confirm that the desired fragment was amplified. Then, the remaining reaction mixture was mixed with an equal amount of phenol/chloroform saturated with TE.

The resulting mixture was centrifuged and the obtained upper layer was mixed with a two-fold volume of cold ethanol and allowed to stand at -80°C for 30 minutes. The resulting mixture was centrifuged to obtain a DNA precipitate.

The DNA precipitate was dissolved in 20 µl of TE [10 mmol/l Tris-HCl, 1 mmol/l EDTA (pH 8.0)] and 5 µl of the solution was subjected to reaction to cleave the DNA with restriction enzymes ClaI and BamHI. DNA fragments were separated by agarose gel electrophoresis and a 1.0 kb DNA fragment containing the UDP-glucose 4-epimerase gene was recovered using Gene Clean II Kit (Funakoshi).

pPAC31 (0.2 µg) was cleaved with restriction enzymes ClaI and BamHI. DNA fragments were separated by agarose gel electrophoresis and a 5.5 kb DNA fragment was recovered in the same manner.

The 1.0 kb fragment and 5.5 kb DNA fragment obtained above were subjected to ligation reaction using a ligation kit (Takara Shuzo Co., Ltd.) at 16°C for 16 hours.

Escherichia coli NM522 was transformed using the ligation mixture according to the known method described above, spread on LB agar medium containing 50 µg/ml ampicillin, and cultured overnight at 30°C.

A plasmid was extracted from a colony of the transformant that grew on the medium according to the known method described above, whereby expression plasmid pGT73 was obtained. The structure of the obtained plasmid was confirmed by digestion with restriction enzymes (Fig. 1) .

### Example 2

### Construction of a Transformant Which Produces UDP-glucose 4-Epimerase Derived from Neisseria gonorrhoeae

Neisseria gonorrhoeae (ATCC 33084) was cultured according to a known method (USP 5,545,553). After the culturing, the chromosomal DNA of the microorganism was prepared by the method described in Current Protocols in Molecular Biology.

On the basis of the nucleotide sequence of the galE gene supposed to encode a protein having UDP-glucose 4-epimerase activity derived from Neisseria gonorrhoeae [Mol. Microbiol., 8., 891 (1993)], DNAs having the nucleotide sequences shown in SEQ ID NOS: 7 and 8 were synthesized using a DNA synthesizer (Model 8905, PerSeptive Biosystems). PCR was carried out using the synthesized DNAs as a set of primers and the chromosomal DNA of Neisseria gonorrhoeae (ATCC 33084) as a template under the same conditions as described above, whereby a ca. 1.0 kb PCR product was obtained.

One-tenth of the resulting reaction mixture was subjected to agarose gel electrophoresis to confirm that the desired fragment was amplified. Then, the remaining reaction mixture was mixed with an equal amount of phenol/chloroform saturated with TE.

The resulting mixture was centrifuged and the obtained upper layer was mixed with a two-fold volume of cold ethanol and allowed to stand at -80°C for 30 minutes. The resulting mixture was centrifuged to obtain a DNA precipitate.

The DNA precipitate was dissolved in 20 µl of TE and 5 µl of the solution was subjected to reaction to cleave the DNA with restriction enzymes ClaI and BamHI. DNA fragments were separated by agarose gel electrophoresis and a 1.0 kb DNA fragment containing the UDP-glucose 4-epimerase gene was recovered using Gene Clean II Kit.

pPAC31 (0.2 µg) was cleaved with restriction enzymes ClaI and BamHI. DNA fragments were separated by agarose gel electrophoresis and a 5.5 kb DNA fragment was recovered in the same manner.

The 1.0 kb fragment and 5.5 kb fragment obtained above were subjected to ligation reaction using a ligation kit at 16°C for 16 hours.

Escherichia coli NM522 was transformed using the ligation mixture according to the known method described above, spread on LB agar medium containing 50 µg/ml ampicillin, and cultured overnight at 30°C.

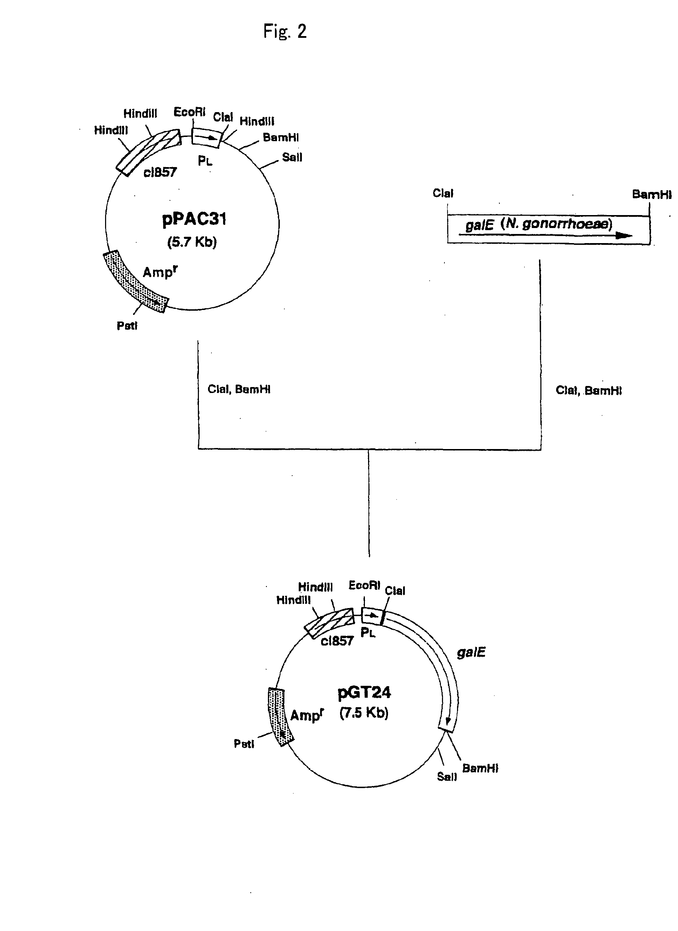

A plasmid was extracted from a colony of the transformant that grew on the medium according to the known method described above, whereby expression plasmid pGT24 was obtained. The structure of the obtained plasmid was confirmed by digestion with restriction enzymes (Fig. 2).

### Example 3

### Construction of a Transformant Which Produces β 1,4-N-Acetylgalactosaminyltransferase Derived from Campylobacter jejuni

Campylobacter jejuni (ATCC 43446) was cultured according to a known method [Microbiology, 144, 2049 (1998)]. After the culturing, the chromosomal DNA of the microorganism was prepared by the method described in Current Protocols in Molecular Biology.

On the basis of the nucleotide sequence of the gene encoding β 1,4-N-acetylgalactosaminyltransferase derived from Campylobacter jejuni (ATCC 43446) [J. Biol. Chem., 275, 3896 (2000)], DNAs having the nucleotide sequences shown in SEQ ID NOS: 9 and 10 were synthesized using a DNA synthesizer (Model 8905, PerSeptive Biosystems). PCR was carried out using the synthesized DNAs as a set of primers and the chromosomal DNA of Campylobacter jejuni (ATCC 43446) as a template under the same conditions as described above, whereby a ca. 1.0 kb DNA fragment was obtained.

One-tenth of the resulting reaction mixture was subjected to agarose gel electrophoresis to confirm that the desired fragment was amplified. Then, the remaining reaction mixture was mixed with an equal amount of phenol/chloroform saturated with TE.

The resulting mixture was centrifuged and the obtained upper layer was mixed with a two-fold volume of cold ethanol and allowed to stand at -80°C for 30 minutes. The resulting mixture was centrifuged to obtain a DNA precipitate.

The DNA precipitate was dissolved in 20 µl of TE and 5 µl of the solution was subjected to reaction to cleave the DNA with restriction enzymes XhoI and BamHI. DNA fragments were separated by agarose gel electrophoresis and a 1.0 kb DNA fragment containing the β 1,4-N-acetylgalactosaminyltransferase gene was recovered using Gene Clean II Kit (Funakoshi).

Separately, PCR was carried out under the same conditions as described above using DNAs having the nucleotide sequences shown in SEQ ID NOS: 11 and 12 as a set of primers and pPAC31 as a template.

One-tenth of the resulting reaction mixture was subjected to agarose gel electrophoresis to confirm that the desired fragment was amplified. Then, the remaining reaction mixture was mixed with an equal amount of phenol/chloroform saturated with TE.

The resulting mixture was centrifuged and the obtained upper layer was mixed with a two-fold volume of cold ethanol and allowed to stand at -80°C for 30 minutes. The resulting mixture was centrifuged to obtain a DNA precipitate.

The DNA precipitate was dissolved in 20 µl of TE and 5 µl of the solution was subjected to reaction to cleave the DNA with restriction enzymes XhoI and EcoRI. DNA fragments were separated by agarose gel electrophoresis and a 0.3 kb DNA fragment containing P_{L} promoter was recovered using Gene Clean II Kit.

pPAC31 (0.2 µg) was cleaved with restriction enzymes EcoRI and BamHI. DNA fragments were separated by agarose gel electrophoresis and a 5.2 kb DNA fragment was recovered in the same manner.

The 1.0 kb fragment, 0.3 kb fragment and 5.2 kb fragment obtained above were subjected to ligation reaction using a ligation kit at 16°C for 16 hours.

Escherichia coli NM522 was transformed using the ligation mixture according to the known method described above, spread on LB agar medium containing 50 µg/ml ampicillin, and cultured overnight at 30°C.

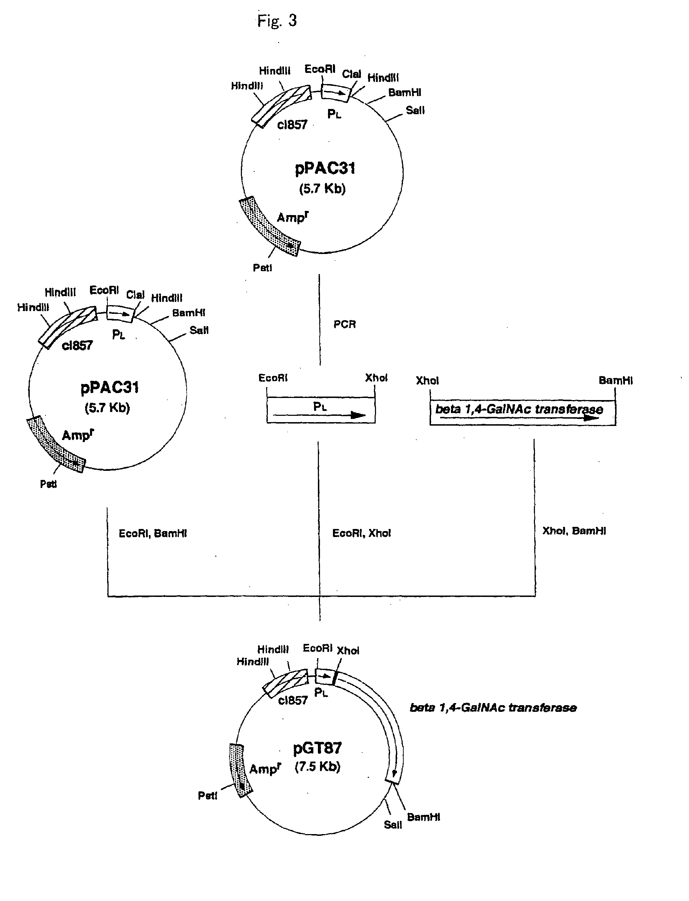

A plasmid was extracted from a colony of the transformant that grew on the medium according to the known method described above, whereby expression plasmid pGT87 was obtained. The structure of the obtained plasmid was confirmed by digestion with restriction enzymes (Fig. 3).

### Example 4

### Construction of a Transformant Which Produces α1,4-N-Acetylgalactosaminyltransferase Derived from Campylobacter jejuni

On the basis of the nucleotide sequence of the gene encoding α1,4-N-acetylgalactosaminyltransferase derived from Campylobacter jejuni (ATCC 43446) [Microbiology, 144, 2049 (1998)], DNAs having the nucleotide sequences shown in SEQ ID NOS: 13 and 14 were synthesized using a DNA synthesizer (Model 8905, PerSeptive Biosystems). PCR was carried out using the synthesized DNAs as a set of primers and the chromosomal DNA of Campylobacter jejuni (ATCC 43446) as a template under the same conditions as in Example 3, whereby a ca. 1.0 kb PCR product was obtained.

One-tenth of the resulting reaction mixture was subjected to agarose gel electrophoresis to confirm that the desired fragment was amplified. Then, the remaining reaction mixture was mixed with an equal amount of phenol/chloroform saturated with TE.

The resulting mixture was centrifuged and the obtained upper layer was mixed with a two-fold volume of cold ethanol and allowed to stand at -80°C for 30 minutes. The resulting mixture was centrifuged to obtain a DNA precipitate.

The DNA precipitate was dissolved in 20 µl of TE and 5 µl of the solution was subjected to reaction to cleave the DNA with restriction enzymes ClaI and BamHI. DNA fragments were separated by agarose gel electrophoresis and a 1.0 kb DNA fragment containing the α1,4-N-acetylgalactosaminyltransferase gene was recovered using Gene Clean II Kit.

pPAC31 (0.2 µg) was cleaved with restriction enzymes ClaI and BamHI. DNA fragments were separated by agarose gel electrophoresis and a 5.5 kb DNA fragment was recovered in the same manner.

The 1.0 kb fragment and 5.5 kb fragment obtained above were subjected to ligation reaction using a ligation kit at 16°C for 16 hours.

Escherichia coli NM522 was transformed using the ligation mixture according to the known method described above, spread on LB agar medium containing 50 µg/ml ampicillin, and cultured overnight at 30°C.

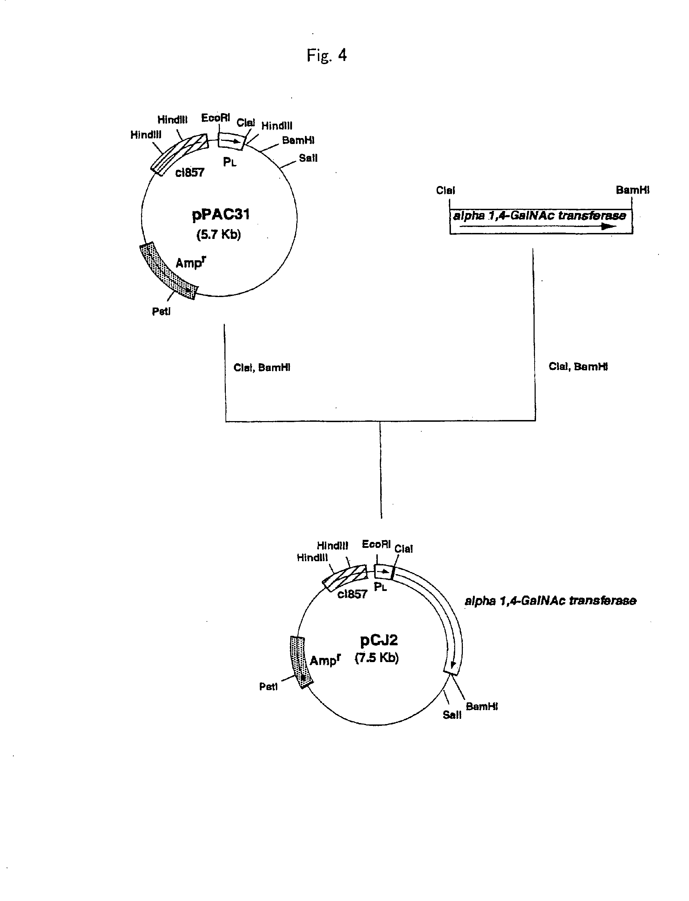

A plasmid was extracted from a colony of the transformant that grew on the medium according to the known method described above, whereby expression plasmid pCJ2 was obtained. The structure of the obtained plasmid was confirmed by digestion with restriction enzymes (Fig. 4).

### Example 5

### Production of UDP-GalNAc (1)

Escherichia coli NM522/pGT73 obtained in Example 1 and Escherichia coli NM522/pNT25 (WO98/12343) producing galactokinase and galactose-1-phosphate uridylyltransferase were respectively inoculated into 125 ml of LB medium containing 50 µg/ml ampicillin in a 1-1 Erlenmeyer flask equipped with baffles, and cultured at 220 rpm at 28°C for 17 hours. Each of the resulting cultures (125 ml) was inoculated into 2.5 1 of TB medium [10 g/l glucose, 12 g/l Bacto-tryptone (Difco), 24 g/l yeast extract (Difco), 2.3 g/l KH₂PO₄ and 12.5 g/l K₂HPO₄ (pH unadjusted)] containing 50 µg/ml ampicillin in a 5-1 fermenter, and cultured at 600 rpm with aeration (2.5 1/min.) at 30°C for 4 hours, followed by further culturing at 40°C for 3 hours. During the culturing, the culture was maintained at pH 7.0 with 28% aqueous ammonia, and glucose was added thereto according to need. The resulting culture was centrifuged to obtain wet cells. Being capable of storage at -20°C as may be required, the wet cells could be used after thawing.

Corynebacterium ammoniagenes ATCC 21170 was inoculated into 25 ml of a liquid medium comprising 50 g/l glucose, 10 g/l polypeptone (Nihon Pharmaceutical Co., Ltd.), 10 g/l yeast extract (Oriental Yeast Co., Ltd.), 5 g/l urea, 5 g/l (NH₄)₂SO₄, 1 g/l KH₂PO₄, 3 g/l K₂HPO₄, 1 g/l MgSO₄·7H₂O, 0.1 g/l CaCl₂·2H₂O, 10 mg/l FeSO₄·7H₂O, 10 mg/l ZnSO₄·7H₂O, 20 mg/l MnSO₄·4-6H₂O, 20 mg/l L-cysteine, 10 mg/l calcium D-pantothenate, 5 mg/l vitamin B1, 5 mg/l nicotinic acid and 30 mg/l biotin (adjusted to pH 7.2 with 10 mol/l NaOH) in a 300-ml Erlenmeyer flask equipped with baffles, and cultured at 220 rpm at 28°C for 24 hours.

The resulting culture (20 ml) was inoculated into 250 ml of a liquid medium having the same composition as above in a 2-1 Erlenmeyer flask equipped with baffles, and cultured at 220 rpm at 28°C for 24 hours. The obtained culture was used as a seed culture.

The seed culture (250 ml) was inoculated into 2.25 1 of a liquid medium comprising 150 g/l glucose, 5 g/l meat extract (Kyokuto Pharmaceutical Ind. Co., Ltd.), 10 g/l KH₂PO₄, 10 g/l K₂HPO₄, 10 g/l MgSO₄·7H₂O, 0.1 g/l CaCl₂·2H₂O, 20 mg/l FeSO₄·7H₂O, 10 mg/l ZnSO₄·7H₂O, 20 mg/l MnSO₄·4-SH₂O (separately sterilized), 15 mg/l β-alanine (separately sterilized), 20 mg/l L-cysteine, 100 mg/l biotin, 2 g/l urea and 5 mg/l vitamin B1 (separately sterilized) (adjusted to pH 7.2 with 10 mol/l NaOH) in a 5-1 fermenter, and cultured at 600 rpm with aeration (2.5 1/mm.) at 32°C for 24 hours. During the culturing, the culture was maintained at pH 6.8 with 28% aqueous ammonia.

The resulting culture was centrifuged to obtain wet cells. Being capable of storage at -20°C as may be required, the wet cells could be used after thawing.

A reaction mixture (30 ml) comprising 50 g/l wet cells of Escherichia coli NM522/pNT25, 50 g/l wet cells of Escherichia coli NM522/pGT73, 150 g/l wet cells of Corynebacterium ammoniagenes ATCC 21170, 100 g/l fructose, 50 g/l N-acetylglucosamine, 10 g/l orotic acid, 25 g/l KH₂PO₄, 5 g/l MgSO₄·7H₂O, 5 g/l phytic acid, 4 g/l Nymeen S-215 and 10 ml/l xylene was put in a 200-ml beaker, and reaction was carried out at 32°C for 27 hours with stirring at 900 rpm with a magnetic stirrer. During the reaction, the reaction mixture was maintained at pH 7.2 with 4 mol/l NaOH, and fructose, N-acetylglucosamine and KH₂PO₄ were added thereto according to need.

After the completion of the reaction, the reaction product was analyzed by HPLC, whereby it was confirmed that 9.6 g/l UDP-GalNAc was formed and accumulated in the reaction mixture.

### Example 6

### Production of UDP-GalNAc (2)

Escherichia coli NM522/pGT24 obtained in Example 2 and Escherichia coli NM522/pNT25 (WO98/12343) were respectively cultured in the same manner as in Example 5 to obtain wet cells. Being capable of storage at -20°C as may be required, the wet cells could be used after thawing.

Corynebacterium ammoniagenes ATCC 21170 was cultured in the same manner as in Example 5 to obtain wet cells. Being capable of storage at -20°C as may be required, the wet cells could be used after thawing.

A reaction mixture (30 ml) comprising 50 g/l wet cells of Bscherichia coli NM522/pNT25, 50 g/l wet cells of Escherichia coli NM522/pGT24, 150 g/l wet cells of Corynebacterium ammoniagenes ATCC 21170, 100 g/l fructose, 50 g/l N-acetylglucosamine, 10 g/l orotic acid, 25 g/l KH₂PO₄, 5 g/l MgSO₄·7H₂O, 5 g/l phytic acid, 4 g/l Nymeen S-215 and 10 ml/l xylene was put in a 200-ml beaker, and reaction was carried out at 32°C for 27 hours with stirring at 900 rpm with a magnetic stirrer. During the reaction, the reaction mixture was maintained at pH 7.2 with 4 mol/l NaOH, and fructose, N-acetylglucosamine and KH₂PO₄ were added thereto according to need.

After the completion of the reaction, the reaction product was analyzed by HPLC, whereby it was confirmed that 5.5 g/l UDP-GalNAc was formed and accumulated in the reaction mixture.

### Example 7

### Production of GM2 Sugar Chain [GalNAc β 1,4 (NeuAc α 2, 3) Gal β 1,4Glc]

Escherichia coli NM522/pGT73 obtained in Example 1, Escherichia coli NM522/pNT25 (WO98/12343 and Escherichia coli NM522/pGT87 obtained in Example 4 were respectively cultured in the same manner as in Example 5 to obtain wet cells. Being capable of storage at -20°C as may be required, the wet cells could be used after thawing.

Corynebacterium ammoniagenes ATCC 21170 was cultured in the same manner as in Example 5 to obtain wet cells. Being capable of storage at -20°C as may be required, the wet cells could be used after thawing.

A reaction mixture (30 ml) comprising 50 g/l wet cells of Escherichia coli NM522/pNT25, 50 g/l wet cells of Escherichia coli NM522/pGT73, 100 g/l wet cells of Escherichia coli NM522/pGT87, 150 g/l wet cells of Corynebacterium ammoniagenes ATCC 21170, 100 g/l fructose, 50 g/l N-acetylglucosamine, 50 g/l UDP-N-acetylglucosamine, 33 g/l 3'-sialyllactose, 25 g/l KH₂PO₄, 5 g/l MgSO₄·7H₂O, 5 g/l phytic acid, 4 g/l Nymeen S-215 and 10 ml/l xylene was put in a 200-ml beaker, and reaction was carried out at 32°C for 27 hours with stirring at 900 rpm with a magnetic stirrer. During the reaction, the reaction mixture was maintained at pH 7.2 with 4 mol/l NaOH, and fructose, N-acetylglucosamine, 3'-sialyllactose and KH₂PO₄ were added thereto according to need.

After the completion of the reaction, the reaction product was analyzed by HPLC, whereby it was confirmed that 7.7 g/l GM2 sugar chain was formed and accumulated in the reaction mixture.

### Example 8

### Production of NOS- α (GalNAc α 1,4 Gal β1,4GlcNAc β1,3Gal β 1,4Glc)

Escherichia coil NM522/pGT24 obtained in Example 2, Bscherichia coli NM522/pNT25 (WO98/12343) and Escherichia coli NM522/pCJ2 obtained in Example 5 were respectively cultured in the same manner as in Example 5 to obtain wet cells. Being capable of storage at -20°C as may be required, the wet cells could be used after thawing.

Corynebacterium ammoniagenes ATCC 21170 was cultured in the same manner as in Example 5 to obtain wet cells. Being capable of storage at -20°C as may be required, the wet cells could be used after thawing.

A reaction mixture (30 ml) comprising 50 g/l wet cells of Escherichia coli NM522/pNT25, 25 g/l wet cells of Escherichia coli NM522/pGT24, 50 g/l wet cells of Escherichia coli NM522/pCJ2, 150 g/l wet cells of Corynebacterium ammoniagenes ATCC 21170, 100 g/l fructose, 100 g/l N-acetylglucosamine, 200 g/l lacto-N-neotetraose, 5 g/l orotic acid, 25 g/l KH₂PO₄, 5 g/l MgSO₄·7H₂O, 5 g/l phytic acid, 4 g/l Nymeen S-215 and 10 ml/l xylene was put in a 200-ml beaker, and reaction was carried out at 32°C for 35 hours with stirring at 900 rpm with a magnetic stirrer. During the reaction, the reaction mixture was maintained at pH 7.2 with 4 mol/l NaOH, and fructose and KH₂PO₄ were added thereto according to need.

After the completion of the reaction, the reaction product was analyzed by HPLC, whereby it was confirmed that 2.4 g/l NOS-α was formed and accumulated in the reaction mixture.

### industrial Applicability

According to the present invention, UDP-GalNAc and an N-acetylgalactosamine-containing carbohydrate can be efficiently produced.

### [Sequence Listing Free Text]

SEQ ID NO: 5 - Description of artificial sequence: synthetic DNA
SEQ ID NO: 6 - Description of artificial sequence: synthetic DNA
SEQ ID NO: 7 - Description of artificial sequence: synthetic DNA
SEQ ID NO: 8 - Description of artificial sequence: synthetic DNA
SEQ ID NO: 9 - Description of artificial sequence: synthetic DNA
SEQ ID NO: 10 - Description of artificial sequence: synthetic DNA
SEQ ID NO: 11 - Description of artificial sequence: synthetic DNA
SEQ ID NO: 12 - Description of artificial sequence: synthetic DNA
SEQ ID NO: 13 - Description of artificial sequence: synthetic DNA
SEQ ID NO: 14 - Description of artificial sequence: synthetic DNA

## Claims

1. A process for producing UDP-N-acetylgalactosamine (hereinafter abbreviated as UDP-GalNAc), which comprises:
allowing an enzyme source and UDP-N-acetylglucosamine (hereinafter abbreviated as UDP-GlcNAc) to be present in
an aqueous medium, said enzyme source being a culture of a transformant which produces a protein having UDP-N-acetylglucosamine 4-epimerase (hereinafter abbreviated as UDP-GlcNAc 4-epimerase) activity or a treated matter of the culture;
allowing UDP-GalNAc to form and accumulate in the aqueous medium; and
recovering UDP-GalNAc from the aqueous medium.

2. A process for producing an N-acetylgalactosamine (hereinafter abbreviated as GalNAc)-containing carbohydrate, which comprises:
allowing an enzyme source, an acceptor carbohydrate, GalNAc transferase and UDP-GlcNAc to be present in an aqueous medium, said enzyme source being a culture of a transformant which produces a protein having UDP-GlcNAc 4-epimerase activity or a treated matter of the culture;
allowing the GalNAc-containing carbohydrate to form and accumulate in the aqueous medium; and
recovering the GalNAc-containing carbohydrate from the aqueous medium.

3. A process for producing UDP-GalNAc, which comprises:
allowing enzyme sources, a precursor of uridine-5'-triphosphate (hereinafter abbreviated as UTP) and a sugar to be present in an aqueous medium, said enzyme sources being a culture of a microorganism having the ability to form UDP-GlcNAc from the precursor of UTP and the sugar or a treated matter of the culture, and a culture of a transformant which produces a protein having UDP-GlcNAc 4-epimerase activity or a treated matter of the culture; allowing UDP-GalNAc to form and accumulate in the aqueous medium; and
recovering UDP-GalNAc from the aqueous medium.

4. A process for producing a GalNAc-containing carbohydrate, which comprises:
allowing enzyme sources, a precursor of UTP, a sugar and an acceptor carbohydrate to be present in an aqueous medium, said enzyme sources being a culture of a microorganism having the ability to form UDP-GlcNAc from the precursor of UTP and the sugar or a treated matter of the culture, GalNAc transferase, and a culture of a transformant which produces a protein having UDP-GlcNAc 4-epimerase activity or a treated matter of the culture; allowing the GalNAc-containing carbohydrate to form and accumulate in the aqueous medium; and
recovering the GalNAc-containing carbohydrate from the aqueous medium.

5. The process according to any of Claims 1 to 4, wherein the treated matter of the culture is concentrated culture, dried culture, cells obtained by centrifuging the culture, a product obtained by subjecting the cells to drying, freeze-drying, treatment with a surfactant, ultrasonication, mechanical friction, treatment with a solvent, enzymatic treatment, protein fractionation or immobilization, or an enzyme preparation obtained by extracting the cells.

6. The process according to Claim 2 or 4, wherein the acceptor carbohydrate is a complex carbohydrate comprising an oligosaccharide having sialic acid, galactose, GalNAc, N-acetylglucosamine, fucose, glucuronic acid or iduronic acid at the nonreducing end.

7. The process according to Claim 6, wherein the oligosaccharide having sialic acid, galactose, GalNAc, N-acetylglucosamine, fucose, glucuronic acid or iduronic acid at the nonreducing end is lactose, N-acetyllactosamine, globotriose, sialyllactose, sialyl N-acetyllactosamine, Lewis X, Lewis a, sialyl Lewis X, sialyl Lewis a, chondroitin sulfate, dermatan sulfate, H type 1 (Fuc α1-2Gal β1-3GlcNAc) or H type 2 (Fuc α1-2Gal β 1-4GlcNAc).

8. The process according to Claim 2 or 4, wherein the acceptor carbohydrate is lactose, N-acetyllactosamine, globotriose, sialyllactose, sialyl N-acetyllactosamine, Lewis X, Lewis a, sialyl Lewis X, sialyl Lewis a, chondroitin sulfate, dermatan sulfate, H type 1 (Fuc α1-2Gal β 1-3GlcNAc) or H type 2 (Fuc α1-2Gal β1-4GlcNAc) .

9. The process according to Claim 3 or 4, wherein the precursor is orotic acid, orotidine, uracil, uridine or uridine-5'-monophosphate.

10. The process according to Claim 3 or 4, wherein the sugar is glucosamine or N-acetylglucosamine.

11. The process according to Claim 3 or 4, wherein the microorganism having the ability to form UDP-GlcNAc from the precursor of UTP and the sugar is one or more microorganisms selected from the group consisting of microorganisms belonging to the genera Escherichia, Corynebacterium and Saccharomyces.

12. The process according to Claim 11, wherein the microorganism belonging to the genus Escherichia is Escherichia coli.

13. The process according to Claim 11, wherein the microorganism belonging to the genus Corynebacterium is Corynebacterium ammoniagenes.

14. The process according to Claim 11, wherein the microorganism belonging to the genus Saccharomyces is Saccharomyces cerevisiae.

15. The process according to any of Claims 1 to 4, wherein the transformant is a transformant obtained by introducing a recombinant DNA into a microorganism.

16. The process according to Claim 15, wherein the microorganism is selected from the group consisting of microorganisms belonging to the genera Escherichia, Corynebacterium and Saccharomyces.

17. The process according to Claim 16, wherein the microorganism belonging to the genus Escherichia is Escherichia coli.

18. The process according to Claim 16, wherein the microorganism belonging to the genus Corynebacterium is Corynebacterium glutamicum.

19. The process according to Claim 16, wherein the microorganism belonging to the genus Saccharomyces is Saccharomyces cerevisiae.

20. The process according to any of Claims 1 to 4, wherein the protein having UDP-GlcNAc 4-epimerase activity is a protein having UDP-GlcNAc 4-epimerase activity which is derived from a microorganism belonging to the genus Bacillus or Neisseria.

21. The process according to Claim 20, wherein the microorganism belonging to the genus Bacillus is selected from the group consisting of Bacillus subtilis, Bacillus megaterium and Bacillus stearothermophilus.

22. The process according to Claim 20, wherein the microorganism belonging to the genus Neisseria is Neisseria gonorrhoeae or Neisseria meningitidis.

23. The process according to any of Claims 1 to 4 and 20 to 22, wherein the protein having UDP-GlcNAc 4-epimerase activity is a protein having the amino acid sequence shown in SEQ ID NO: 1 or 2.

24. The process according to any of Claims 1 to 4 and 20 to 22, wherein the protein having UDP-GlcNAc 4-epimerase activity is a protein consisting of an amino acid sequence wherein one or more amino acid residues are deleted, substituted, inserted or added in the amino acid sequence shown in SEQ ID NO: 1 or 2 and having UDP-GlcNAc 4-epimerase activity.

25. The process according to any of Claims 1 to 4 and 20 to 22, wherein the protein having UDP-GlcNAc 4-epimerase activity is a protein consisting of an amino acid sequence having 50% or more homology to the amino acid sequence shown in SEQ ID NO: 1 or 2.

26. The process according to Claim 15, wherein the recombinant DNA comprises DNA encoding a protein having UDP-GlcNAc 4-epimerase activity.

27. The process according to Claim 15, wherein the recombinant DNA comprises DNA encoding UDP-glucose 4-epimerase (hereinafter abbreviated as galE protein) having UDP-GlcNAc 4-epimerase activity.

28. The process according to Claim 27, wherein the galE protein is derived from a microorganism belonging to the genus Bacillus or Neisseria.

29. The process according to Claim 28, wherein the microorganism belonging to the genus Bacillus is selected from the group consisting of Bacillus subtilis, Bacillus megaterium and Bacillus stearothermophilus.

30. The process according to Claim 28, wherein the microorganism belonging to the genus Neisseria is Neisseria gonorrhoeae or Neisseria meningitidis.

31. The process according to Claim 15, wherein the recombinant DNA comprises DNA encoding a protein having the amino acid sequence shown in SEQ ID NO: 1 or 2.

32. The process according to Claim 15, wherein the recombinant DNA comprises DNA encoding a protein consisting of an amino acid sequence wherein one or more amino acid residues are deleted, substituted, inserted or added in the amino acid sequence shown in SEQ ID NO: 1 or 2 and having UDP-GlcNAc 4-epimerase activity.

33. The process according to Claim 15, wherein the recombinant DNA comprises DNA encoding a protein consisting of an amino acid sequence which has 50% or more homology to the amino acid sequence shown in SEQ ID NO: 1 or 2 and having UDP-GlcNAc 4-epimerase activity.

34. The process according to Claim 15, wherein the recombinant DNA comprises DNA having the nucleotide sequence shown in SEQ ID NO: 3 or 4.

35. The process according to Claim 15, wherein the recombinant DNA comprises DNA which hybridizes with DNA consisting of the nucleotide sequence shown in SEQ ID NO: 3 or 4 under stringent conditions and which encodes a protein having UDP-GlcNAc 4-epimerase activity.
